# EUROPÄISCHE PATENTSCHRIFT

(11) **EP 3 793 492 B1**
(45) Veröffentlichungstag und Bekanntmachung des Hinweises auf die Patenterteilung: **15.07.2026**
(21) Anmeldenummer: 19725154.9
(22) Anmeldetag: 17.05.2019
(51) Int. Cl.: A61F 5/01, A61F 13/06

(54) **STABILISIERUNGSSTAB FÜR EIN ORTHOPÄDISCHES HILFSMITTEL**
STABILIZING ROD FOR AN ORTHOPAEDIC AID
BARRE STABITLISATRICE DESTINÉE À UN AUXILIAIRE ORTHOPÉDIQUE

(30) Priorität: 17.05.2018 DE 102018207727
(43) Veröffentlichungstag der Anmeldung: 24.03.2021
(73) Patentinhaber: Bauerfeind AG, 07937 Zeulenroda-Triebes (DE)
(72) Erfinder: BAUERFEIND, Hans B., 07937 Zeulenroda-Triebes (DE)
(74) Vertreter: Wohlfahrt, Jan Günther
(86) Internationale Anmeldenummer: PCT/EP2019/062833
(87) Internationale Veröffentlichungsnummer: WO 2019/219925

(56) Entgegenhaltungen:
- WO-A1-2011/035885
- DE-A1- 4 412 765
- US-A- 1 188 355
- US-A1- 2012 004 584
- US-A1- 2017 297 278
- US-A1- 2017 318 872
- US-B1- 8 784 349

## Beschreibung

Die vorliegende Erfindung betrifft Stabilisierungsstäbe für ein orthopädisches Hilfsmittel, insbesondere Kniebandagen und orthopädisches Hilfsmittel, umfassend mindestens einen erfindungsgemäßen Stabilisierungsstab.

Der Einsatz von Stabilisierungsstäben in orthopädischen Hilfsmitteln ist bekannt. Kniegelenksbandagen, kurz Kniebandagen, mit seitlichen Stabilisierungsstäben sind aus der WO 2011/035885 A1 und der DE 3637 879 A1 bekannt. Dabei werden Stabilisierungsstäbe aus Metall verwendet, die durch den Aufbau als Federbandstäbe, also flachgepressten Windungen einer Schraubenfeder, biegsam sind. Da solche Stabilisierungsstäbe jedoch häufig an das Textil des orthopädischen Hilfsmittels angeschweißt werden, müssen zumindest Teilbereiche der metallischen Federbandstäbe mit einem anschweißbaren Kunststoff kaschiert werden. Darüber hinaus kann das Metall das anliegende Textil zerstören. Die eingesetzten flachen Spiralfedern sind dazu über den ganzen Verlauf der Feder gleich elastisch und der Grad der Einbeugung kann nicht limitiert werden. Ferner sind Stabilisierungsstäbe aus DE 4412765 A1, US 1188355 A, US 8784349 B1 und US 2017/297278 A1 bekannt.

Das der vorliegenden Erfindung zugrunde liegende technische Problem ist die Bereitstellung von verbesserten Stabilisierungsstäben für ein orthopädisches Hilfsmittel, insbesondere Kniebandagen, die mindestens so gut funktionieren wie die Stabilisierungsstäbe aus dem Stand der Technik, aber dabei einfacher und billiger zu produzieren sind, gut verschweißbar sind, leichter sind und oder das anliegende Textil schonen.

Die vorliegende Erfindung löst dieses technische Problem durch einen Stabilisierungsstab nach Anspruch 1.

Insbesondere löst die vorliegende Erfindung das ihr zugrunde liegende technische Problem durch einen Stabilisierungsstab für ein orthopädisches Hilfsmittel, insbesondere für eine Kniebandage, wobei der Stabilisierungsstab einen biegsamen Abschnitt aufweist, wobei der biegsame Abschnitt eine Gitterbandstruktur aufweist.

Bevorzugt weist der biegsame Abschnitt mindestens eine Gitterbandstruktur auf.

Bevorzugt erstreckt sich die Gitterbandstruktur über die Länge des Stabilisierungsstabs.

Im Zusammenhang mit der vorliegenden Erfindung wird unter einer Gitterbandstruktur ein Netzband oder das Gitterband verstanden, also eine gitterförmige Struktur mit Zwischenräumen. Bevorzugt hat die Gitterbandstruktur eine mehreckige, insbesondere karoförmige oder rautenförmige Struktur, das heißt die durch das Gitter gebildeten Zwischenräume sind karoförmig oder rautenförmig, beispielsweise wabenartig.

Es sind aber auch andere Ausformungen möglich, zum Beispiel rechteckig, rund, oval, ellipsenförmig, dreieckig, sechseckig, achteckig oder vieleckig.

Bei einer eckigen Ausformung der einzelnen Strukturelemente der Gitterbandstruktur, insbesondere bei einer karoförmigen oder rautenförmigen Struktur ist der Winkel der Ecken frei wählbar. Durch die Wahl der Winkelgröße kann in vorteilhafter Weise die Flexibilität des Stabilisierungsstabs beeinflusst werden. Beispielsweise können die Ecken in der Vorzugsrichtung der Verformung des Stabilisierungsstabs einen Winkel von mindestens 30° und höchstens 55° haben.

Bevorzugt haben insbesondere bei einer Rautenform jeweils zwei gegenüber liegende Ecken den gleichen Winkel, zwei nebeneinander liegende Ecken jedoch einen unterschiedlichen Winkel. Dabei kann der Unterschied zwischen den Winkeln mindestens 0,5° betragen.

Im Zusammenhang mit der vorliegenden Erfindung wird unter dem Winkel einer Ecke der Winkel verstanden, der bei der Ecke im ungedehnten, ungestreckten und ungebogenen Zustand vorliegt.

Insbesondere bei rautenförmigen oder dreieckigen Strukturen kann eine Ecke oder können zwei oder mehrere Ecken als Scharnier ausgebildet sein, also so biegsam, dass der Winkel der Ecke unterschiedlich groß sein kann.

Bei einer dreieckigen Ausführungsform der Strukturelemente ist bevorzugt die Hypotenuse eines Dreiecks mit der Spitze eines anderen Dreiecks verbunden.

Der Federeffekt bei einer dreieckigen Ausführungsform der Strukturelemente entsteht durch eine konvexe oder konkave Verformung der Hypotenuse. Durch die Verformung nähern sich die an der Hypotenuse angrenzenden Ecken an, sodass das Strukturelement bei der Verformung schmaler wird.

Bei einer runden Ausführungsform der Strukturelemente kann der Radius frei gewählt werden, ebenso der Krümmungsbereich bei einer ovalen oder ellipsoiden Ausführungsform der Strukturelemente.

Die Strukturelemente können auch eine längliche Form aufweisen, beispielsweise bananenartig. Insbesondere wenn die Struktur einen dünnen, biegsamen Stab neben oder in der Gitterbandstruktur aufweist oder wenn mehr als eine Gitterbandstruktur vorgesehen ist, sind die einzelnen Strukturelemente der Gitterbandstruktur bevorzugt dreieckig oder viereckig und/oder weisen nicht mehr als vier Ecken auf, da beispielsweise Sechsecke oder Achtecke weniger biegsam sind.

Bei der erfindungsgemäßen Gitterbandstruktur liegen mehrere der durch die gitterförmige Struktur, also dem Gitter gebildeten Zwischenräume in Längsrichtung des Stabilisierungsstabs hintereinander. Die Gitterbandstruktur umfasst also mehrere durch das Gitter gebildete in Reihe liegende Zwischenräume.

Der Stabilisierungsstab ist einstückig und besteht aus Kunststoff.

Der Stabilisierungsstab kann eine Beschichtung aufweisen.

Auch können die durch die Gitterbandstruktur gebildeten Zwischenräume mit einem weiteren Material gefüllt sein, insbesondere einem weicheren Material wie zum Beispiel einem Schaum. Dadurch kann auch die Biegsamkeit des Stabilisierungsstabes beeinflusst werden.

Auch über die Wahl der Größe der Zwischenräume kann in vorteilhafter Weise die Biegsamkeit des entsprechenden Abschnitts oder des gesamten Stabilisierungsstabs eingestellt werden. Auch durch das Verhältnis sowie durch die Graduierung der Innenwinkel der bevorzugt rautenförmigen Zwischenräume kann die Biegsamkeit beeinflusst werden.

Auch durch die Länge und Positionierung des biegsamen Abschnitts kann in vorteilhafter Weise Lage und Stärke der Biegsamkeit des Stabilisierungsstabes bestimmt werden.

Eine weitere Einstellmöglichkeit der Biegsamkeit ergibt sich durch die Wahl der Dicke und des Querschnitts eines fakultativen dünnen, biegsamen Stabs.

Der Stabilisierungsstab umfasst einen dünnen biegsamen Stab und mindestens eine Gitterbandstruktur. Bevorzugt wird der Stabilisierungsstab aus einem dünnen biegsamen Stab und mindestens einer Gitterbandstruktur gebildet. Der dünne biegsame Stab kann am Rand der Gitterbandstruktur liegen oder durch die Gitterbandstruktur mittig oder seitlich versetzt hindurchführen.

In einer besonderen Ausführungsform kann der dünne biegsame Stab eine Führung, beispielsweise einem Führungskanal aufweisen. Beispielsweise kann der dünne biegsame Stab hohl sein, insbesondere als Röhre ausgeführt sein. Dadurch kann in dem Stab eine Schnur oder ähnliches geführt werden oder es kann sich in dem Stab eine Seele befinden, beispielsweise eine Metalseele, die die Stabilität des Stabilisierungsstabs erhöht oder seine Federkraft verstärkt.

Bevorzugt wird der biegsame Abschnitt des Stabilisierungsstabs, insbesondere der Stabilisierungsstab aus einem dünnen biegsamen Stab und mindestens einer an diesem Stab verlaufenden Gitterbandstruktur gebildet.

Bevorzugt wird der biegsame Abschnitt des Stabilisierungsstabs, insbesondere der Stabilisierungsstab aus einem dünnen biegsamen Stab und einer an diesem Stab verlaufenden Gitterbandstruktur gebildet.

Der Stabilisierungsstab kann aber auch mehrere Gitterbandstrukturen aufweisen, beispielsweise zwei oder drei Gitterbandstrukturen. Insbesondere kann der Stabilisierungsstab mehrere, beispielsweise zwei nebeneinander angeordnete Gitterbandstrukturen aufweisen, so dass also mehrere, beispielsweise zwei durch Gitter gebildete Reihen von Zwischenräume sich in Längsrichtung des Stabilisierungsstabs erstrecken.

Ebenso bevorzugt wird daher der biegsame Abschnitt des Stabilisierungsstabs, insbesondere der Stabilisierungsstab aus zwei Gitterbandstrukturen gebildet.

Bevorzugt wird daher der biegsame Abschnitt des Stabilisierungsstabs, insbesondere der Stabilisierungsstab aus zwei Gitterbandstrukturen und einem zwischen diesen verlaufenden dünnen biegsamen Stab gebildet.

Im Folgenden werden auch Beispiele beschrieben, die keinen dünnen biegsamen Stab aufweisen. Diese Beispiele fallen nicht in den Schutzbereich der Ansprüche und werden lediglich zur Information offenbart.

Alternativ wird der biegsame Abschnitt des Stabilisierungsstabs ohne dünnen biegsamen Stab und nur aus mindestens einer Gitterbandstruktur gebildet. Bevorzugt wird der biegsame Abschnitt des Stabilisierungsstabs ohne dünnen biegsamen Stab und nur aus einer Gitterbandstruktur gebildet. Bevorzugt wird der biegsame Abschnitt des Stabilisierungsstabs ohne dünnen biegsamen Stab und nur aus mindestens zwei, bevorzugt zwei Gitterbandstrukturen gebildet. Bevorzugt ist ein Stabilisierungsstab, bei dem sich eine, insbesondere rautenförmige Gitterbandstruktur oder zwei parallele, insbesondere rautenförmige Gitterbandstrukturen über die Länge des Stabilisierungsstabs erstrecken.

Bevorzugt ist ein Stabilisierungsstab, bei dem sich zwei parallele, insbesondere rautenförmige Gitterbandstrukturen über die Länge des Stabilisierungsstabs erstrecken und zwischen diesen Gitterbandstrukturen ein dünner, biegsamer Stab verläuft.

Beschrieben sind also insbesondere vier alternative bevorzugte Ausführungsformen des biegsamen Abschnitts des Stabilisierungsstabs: 1. Ein Gitterband ohne dünnen biegsamen Stab. 2. Ein Gitterband mit zugeordnetem dünnem biegsamen Stab. 3. Mindestens zwei Gitterbänder, insbesondere zwei Gitterbänder ohne dünnen biegsamen Stab. 4. Mindestens zwei Gitterbänder, insbesondere zwei Gitterbänder mit zugeordnetem dünnem biegsamen Stab.

Es zeigte sich überraschender Weise weiter, dass durch eine Gitterbandstruktur eine gute und ausreichende Biegsamkeit des Stabilisierungsstabs erreicht werden kann, ohne dass der Stabilisierungsstab als Federbandstab ausgebildet sein muss. Da kein Federbandstab, insbesondere durchgehender Federbandstab notwendig ist, kann in vorteilhafter Weise auf Metall und/oder das kreisförmig überlappende Federband verzichtet werden, wodurch der Stabilisierungsstab das anliegende Textil schont. Auch kann der Stabilisierungsstab in vorteilhafter Weise einstückig und aus Kunststoff hergestellt werden. Durch den Verzicht auf einen Federbandstab aus Metall wird sowohl die Gefahr des Brechens des Stabes mit der verbundenen Verletzungsgefahr durch scharfkantige Metallstücke als auch die mögliche Geräuschentwicklung, wie Knarzen oder Quietschen reduziert. Auch ein durch Schweiß verursachtes Rosten kann verhindert werden. Der erfindungsgemäße Stabilisierungsstab ist durch die Gitterbandstruktur und die bevorzugte Ausgestaltung aus Kunststoff auch besonders leicht. Ein bevorzugter Kunststoff ist ein thermoplastischer Kunststoff, beispielsweise Polyurethan (TPU). Der Stabilisierungsstab kann in vorteilhafter und einfacher Weise durch Spritzguss hergestellt werden.

Durch die Position des biegsamen Abschnitts entlang der Länge des Stabilisierungsstabs kann der biegsame Bereich des Stabilisierungsstabs bestimmt werden.

Wenn der biegsame Abschnitt sich bevorzugt über die gesamte Länge des Stabilisierungsstabs erstreckt, kann der biegsame oder besonders biegsame Bereich des Stabilisierungsstabs durch die Materialdicke der jeweiligen Abschnitte bestimmt werden, indem der besonders biegsame Abschnitt eine geringere Materialdicke aufweist. Alternativ oder zusätzlich kann die Biegsamkeit in diesem Bereich auch durch ein anderes Verhältnis der Innenwinkel der Zwischenräume vergrößert werden, insbesondere bei rautenförmigen Zwischenräumen.

Der Stabilisierungsstab ist einstückig und besteht aus einem Kunststoff, auch aus einem gummiartigen Kunststoff.

Dem Fachmann sind geeignete Kunststoffe bekannt, beispielsweise thermoplastisches Polyurethan (TPU), Polypropylen (PP) oder Polyethylen (PE). Es ist aber auch die Verwendung rigider Materialien möglich, beispielsweise zur Definition einer Vorzugsrichtung oder zur Ausgestaltung eines Scharniers.

Auch kann das Grundmaterial beschichtet werden, beispielsweise um eine bessere Haftung des Stabs an das Bandagenmaterial zu erreichen.

Bevorzugt ist der Stabilisierungsstab insbesondere in Längsrichtung kaum dehn- und/oder stauchbar, insbesondere auch wenn der bevorzugte dünne und biegsame Stab vorhanden ist.

Bevorzugt weist der erste und/oder der zweite Abschnitt des Stabilisierungsstabs, insbesondere der erste Abschnitt des Stabilisierungsstabs ein Griffstück auf. Bevorzugt ist das Griffstück als Öse ausgebildet. Bevorzugt weist die Öse an ihrer dem des Stabilisierungsstab abgewandten Seite eine Verdickung auf. Als Alternative zu einer Öse kann das mindestens eine Griffstück auch Noppen auf dem Stabilisierungsstab aufweisen, die cin Greifen des Stabs beim An- und Ausziehen vereinfachen.

Der Stabilisierungsstab wird hierbei mit doppelter Wirkung ausgenutzt, nämlich einerseits zur Stabilisierung des Kniegelenks und andererseits als Anziehhilfe und Ausziehilfe, wozu der Stabilisierungsstab mit mindestens einem Griffstück versehen ist, das leicht erfassbar ist und einen auf ihn ausgeübten Zug direkt auf das Bandagenmaterial überträgt. Es kann am oberen und/oder unteren Ende des Stabilisierungsstabs ausgebildet sein. Beim Zug an dem Griffstück wird dieser auf diese Weise über die gesamte Länge der Bandage in diese eingeleitet. Das Griffstück wird zweckmäßig als Öse ausgebildet, bei dem der Durchgang durch sein Loch etwa rechtwinkelig zum Bandagenmaterial liegt. Bei einer solchen Ausbildung des Griffstücks kann dieses mit einem Finger direkt erfasst werden, der dabei die Öse durchsetzt und den Zug auf diese Weise bequem auf die Bandage überträgt. Das Erfassen der Öse lässt sich weiterhin dadurch erleichtern, dass diese an ihrer dem Stabilisierungsstab abgewandten Seite eine Verdickung aufweist. Auf diese Weise kann die Bandage zum An- und ausziehen leichter mit den Fingern gegriffen werden.

Es zeigte sich überraschender Weise, dass der Stabilisierungsstab trotz der Gitternetzstruktur dennoch stabil genug ist um auch als Anziehhilfe verwendet werden zu können.

Bevorzugt handelt es sich bei dem erfindungsgemäßen Stabilisierungsstab um einen Stabilisierungsstab für eine Bandage. Bevorzugt handelt es sich bei dem erfindungsgemäßen Stabilisierungsstab um einen Stabilisierungsstab für eine Kniegelenkbandage.

Die vorliegende Erfindung betrifft auch die Verwendung eines erfindungsgemäßen Stabilisierungsstabs in einem orthopädischen Hilfsmittel, insbesondere einer Bandage, bevorzugt einer Kniegelenkbandage umfassend einen Stabilisierungsstab nach einem der vorhergehenden Ansprüche.

Die vorliegende Erfindung betrifft auch ein orthopädisches Hilfsmittel, umfassend einen erfindungsgemäßen Stabilisierungsstab. Bevorzugt ist das orthopädische Hilfsmittel eine Bandage. Besonders bevorzugt ist das orthopädische Hilfsmittel eine Kniebandage beziehungsweise Kniegelenksbandage. Bevorzugt ist der Grundkörper der Kniebandage beziehungsweise Kniegelenksbandage aus einem Textil, insbesondere einem Gestrick oder Gewirk gebildet. Geeignete Kniebandagen beziehungsweise Kniegelenksbandagen und deren Grundkörper sind dem Fachmann bekannt.

Die vorliegende Erfindung betrifft daher auch eine Kniegelenksbandage, umfassend einen erfindungsgemäßen Stabilisierungsstab.

Bevorzugt ist eine Kniegelenksbandage, wobei sich der biegsame Abschnitt des Stabilisierungsstabs im angelegten Zustand der Kniegelenksbandage auf der Höhe des Knies befindet.

Bevorzugt weist die Kniegelenksbandage zwei Stabilisierungsstäbe auf, insbesondere zwei erfindungsgemäße Stabilisierungsstäbe.

Bevorzugt ist eine Kniegelenksbandage, wobei die Kniegelenksbandage zwei Stabilisierungsstäbe, insbesondere zwei erfindungsgemäße Stabilisierungsstäbe, aufweist, wobei sich die Stabilisierungsstäbe über die Länge der Kniegelenksbandage erstrecken. Bevorzugt erstecken sich die Stabilisierungsstäbe seitlich des Knies über die Länge der Kniegelenksbandage.

Bevorzugt ist eine Kniegelenksbandage, wobei sich jeweils der biegsame Abschnitt der Stabilisierungsstäbe im angelegten Zustand der Kniegelenksbandage auf der Höhe des Knies befindet.

Bevorzugt ist der mindestens eine Stabilisierungsstab in einer an der Bandage angeordneten Tasche eingebettet. Bevorzugt ist ein Teilbereich des mindestens einen Stabilisierungsstabs mit dem Textil der Bandage und/oder der Tasche verschweißt.

Bevorzugt ist eine aus elastischem Material, insbesondere einem Textil bestehende Bandage, insbesondere Kniegelenksbandage, die auf mindestens einer Seite mit einem erfindungsgemäßen sich über die Länge der Bandage erstreckenden Stabilisierungsstab versehen ist, wobei der Stabilisierungsstab mit einem oder zwei Griffstücken versehen und in eine an der Bandage angeordneten Tasche eingebettet ist, die über Randzonen und an ihrem oberhalb der Kniescheibe angeordneten Ende mit dem Material der Bandage fest verbunden ist. Bevorzugt ist der Stabilisierungsstab im Wesentlichen durchgehend mit dem Material der Bandage verschweißt oder ganz oder teilweise formschlüssig über eine Tasche oder über mindestens einer Öse oder Schlaufe mit dem Material der Bandage verbunden. Bevorzugt ist das Griffstück als Öse ausgebildet, bei dem der Durchgang durch sein Loch etwa rechtwinklig zum Bandagenmaterial liegt. Bevorzugt weist die an ihrer dem Stabilisierungsstab abgewandten Seite eine Verdickung auf.

Bevorzugt weist die Kniegelenksbandage ein der Kniescheibe zugeordnetes Polster auf.

Weitere bevorzugte Ausführungsformen ergeben sich aus den Unteransprüchen, den Beispielen und den Figuren.
- Figur 1: zeigt einen erfindungsgemäßen Stabilisierungsstab;
- Figur 2: zeigt eine alternative Ausführungsform des erfindungsgemäßen Stabilisierungsstabs;
- Figur 3: zeigt eine erfindungsgemäße Kniegelenksbandage mit zwei Stabilisierungsstäben aus Figur 2;
- Figur 4: zeigt ein Beispiel eines nicht in den Schutzbereich der Ansprüche fallenden Stabilisierungsstabs;
- Figur 5: zeigt verschiedene Ausführungsformen der Gitterbandstruktur,

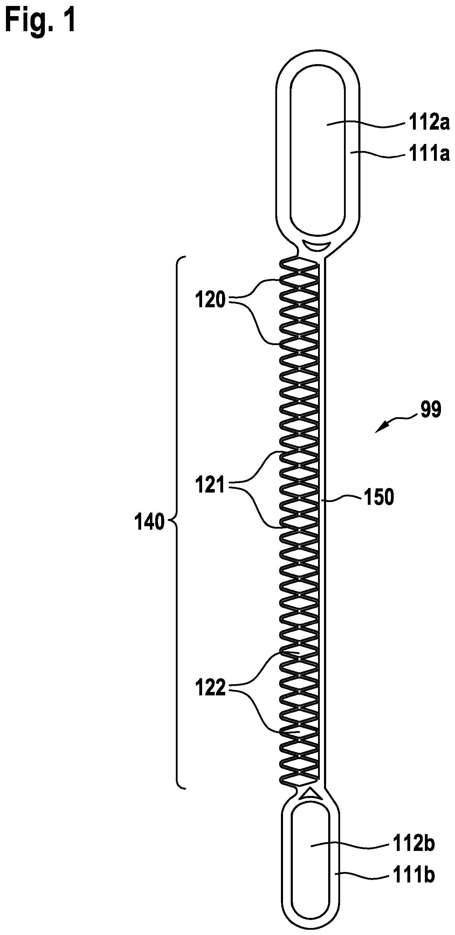

Figur 1 zeigt einen erfindungsgemäßen Stabilisierungsstab 99. Der Stabilisierungsstab 99 ist einstückig ausgebildet und aus einem Kunststoff geformt.

Der biegsame Abschnitt 140 des Stabilisierungsstabs 99 wird aus einer Gitterbandstruktur 120 und einem an der Gitterbandstruktur liegenden dünnen biegsamen Stab 150 gebildet. Die Gitterbandstruktur 120 wird aus einem Gitter 121 gebildet, das rautenförmige Zwischenräume 122 umschließt. Durch die Gitterbandstruktur 120 ist der Stabilisierungsstab 99 biegsam und leicht. Durch den dünnen biegsamen Stab 150 wird eine Dehnbarkeit beziehungsweise Stauchbarkeit in Längsrichtung des Stabilisierungsstabs 99 eingeschränkt, sodass der Stabilisierungsstab 99 in vorteilhafter Weise auch als Anzieh- und Ausziehhilfe für eine elastische Bandage, beispielsweise für eine Kniebandage verwendet werden kann.

An den Enden weist der Stabilisierungsstabs 99 dafür jeweils ein Griffstück 111a/111b auf, die Eingriffslöcher 112a/112b ausbilden. Die Griffstücke 111a/111b sind Bestandteil des Stabilisierungsstabs 99 und einstückig aus dessen Material gebildet.

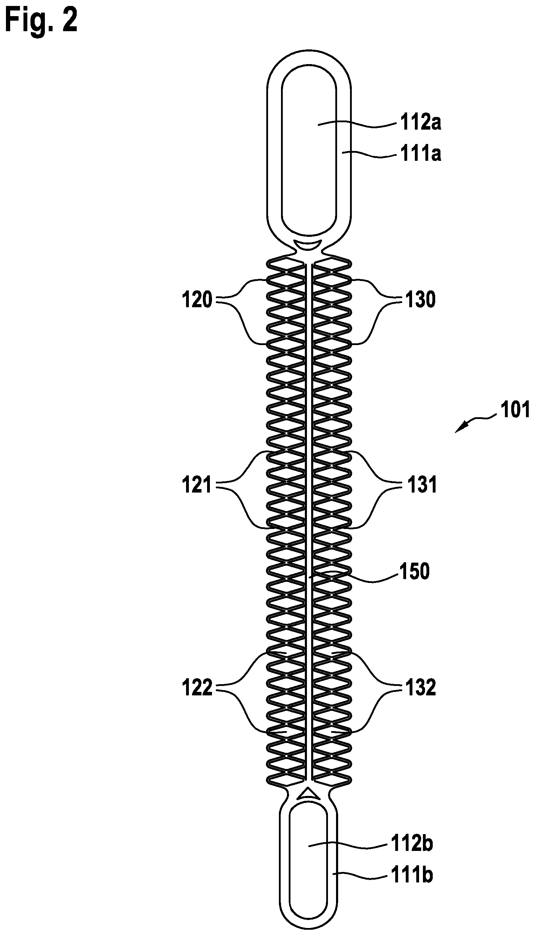

Figur 2 zeigt eine weitere Ausführungsform eines erfindungsgemäßen Stabilisierungsstabs 101. Der Stabilisierungsstab 101 ist einstückig ausgebildet und aus einem Kunststoff geformt.

Wie in Figur 1 umfasst der Stabilisierungsstab 101 eine Gitterbandstruktur 120 und einen dünnen biegsamen Stab 150. Zusätzlich weist der Stabilisierungsstab 101 neben der ersten Gitterbandstruktur 120 mit den Gitterelementen 121 und den rautenförmigen Zwischenräumen 122 eine zweite Gitterbandstruktur 130 auf, die ebenfalls durch die Gitter 131 gebildete rautenförmige Zwischenräume 132 aufweist. Der dünne biegsame Stab 150 verläuft zwischen den beiden Gitterbandstrukturen 120/130. Der dünne biegsame Stab 150 kann beispielsweise auch hohl sein oder einen Führungskanal aufweisen um eine Schnur oder eine Metalseele hindurchzuführen.

In dieser Ausführungsform ist der Stabilisierungsstab breiter aber dennoch gut biegsam. An den Enden weist der Stabilisierungsstabs 101 wieder jeweils ein Griffstück 111a/111b auf, die Eingriffslöcher 112a/b ausbilden. Die Griffstücke 111a/111b sind Bestandteil des Stabilisierungsstabs 101 und einstückig aus dessen Material gebildet.

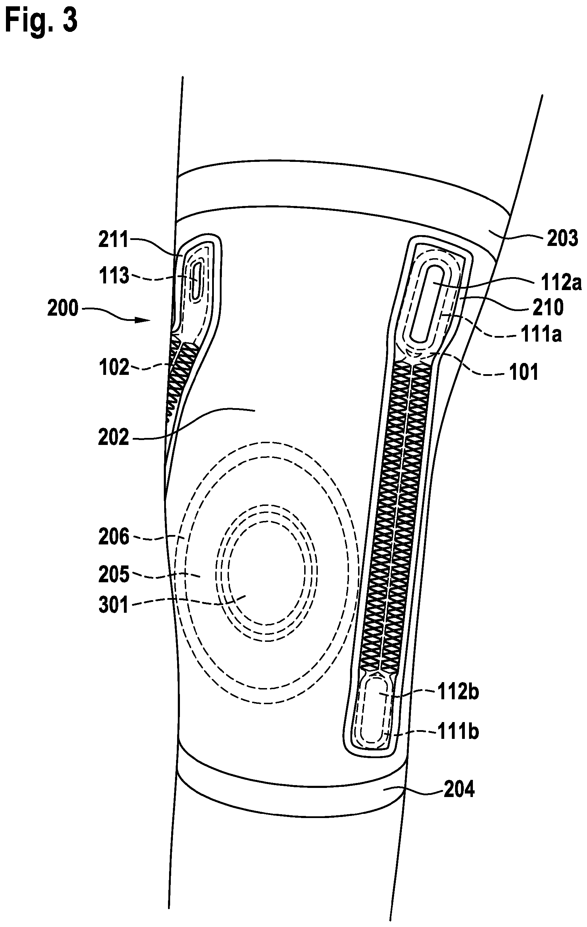

Die in der Figur 3 dargestellte Kniegelenkbandage 200 besteht aus dem aus elastischem Textilmaterial bestehenden Strumpf 202 und sie ist mit den beiden Rändern 203 und 204 an ihren beiden Enden versehen, die zur Rutschsicherung der Bandage 200 beitragen. Außerdem sind diese Ränder 203 und 204 aus einem Material hergestellt, das eine geringere Spannung als der Strumpf 202 aufweist, um das Bein des Trägers an den betreffenden Stellen nur wenig einzuschnüren. Auf der Vorderseite des Kniegelenks ist in dem Strumpf 202 eine Profileinlage mit dem Polster 205 eingearbeitet, die zum Beispiel aus Schaumstoff oder Silikon bestehen kann und eine erhebliche Elastizität besitzt. Das Polster 205 ist auf der Innenseite des Strumpfes 202 durch einen Überzug abgedeckt, der an seinen Rändern 206 mit dem Strumpf 202 verbunden ist, beispielsweise durch Kleben. Das Polster 205 lässt in ihrem mittleren Teil einen Bereich frei, in dem etwa die Kniescheibe 301 hineinpasst. Die Kniescheibe 301 wird somit von dem Polster 205 umfasst. Insoweit handelt es sich um eine in bekannter Weise gestaltete Kniegelenkbandage. Neben dem Polster 205 ist die Bandage 200 mit zwei erfindungsgemäßen Stabilisierungsstäben 101 und 102 versehen, die sich im Wesentlichen über die gesamte Länge der Bandage 200 erstrecken und die dafür sorgen, dass sich die am Bein angelegte Bandage 200 nicht hinsichtlich ihrer Längsrichtung zusammenziehen kann. Bei diesen Stabilisierungsstäben 101 und 102 handelt es sich um Stabilisierungsstäbe, wie in Figur 2 gezeigt. Jeder der beiden Stabilisierungsstäbe 101 und 102 ist in eine auf die Bandage 200 mittels der Randzone 210 bzw. 211 an das Material der Bandage 200 angeklebte Tasche aufgenommen. Je nach gewünschter Stabilisierungsintensität kann die Bandage 200 auch nur mit einem Stabilisierungsstab versehen sein. Jeder der beiden Stabilisierungsstäbe 101 und 102 weist an den Enden ein eine Öse 112a/112b bzw. 113 enthaltendes Griffstück 111a/111b auf, welches das Erfassen der Bandage 200 bei ihrem Anziehen und Hochziehen beziehungsweise Ausziehen und Herunterziehen entlang des Beines mit dem Finger ermöglicht und damit das An- bzw. Ausziehen der Bandage 200 erleichtert, da die Bandage 200 bei einem entsprechenden Zug auf die Griffstücke 111a/111b von diesen und dem Stabilisierungsstab 101, 102 insgesamt mitgenommen wird, wodurch es ohne weiteres möglich ist, die Bandage 200 glatt über den Fuß, die Wade und das Knie in ihre endgültige Lage zu ziehen. Die in den Taschen enthaltenen Stabilisierungsstäbe 101 und 102 werden dadurch satt von den betreffenden Taschen umfasst, dass deren Randzone 210 und 211 jeweils als schmaler umlaufener Streifen ausgebildet ist, der direkt mit dem Material der Bandage 200 verbunden ist, zum Beispiel durch verschweißen oder durch verkleben.

Auf die besondere erfindungsgemäß bevorzugte Gestaltung der Stabilisierungsstäbe 101, 102 wird in Figur 2 näher eingegangen.

Der biegsame Abschnitt der Stabilisierungsstäbe 101, 102 befindet sich dabei auf Höhe der Kniescheibe 301. Somit wird beim Beugen des Knies auch genau der biegsame Abschnitt gebogen.

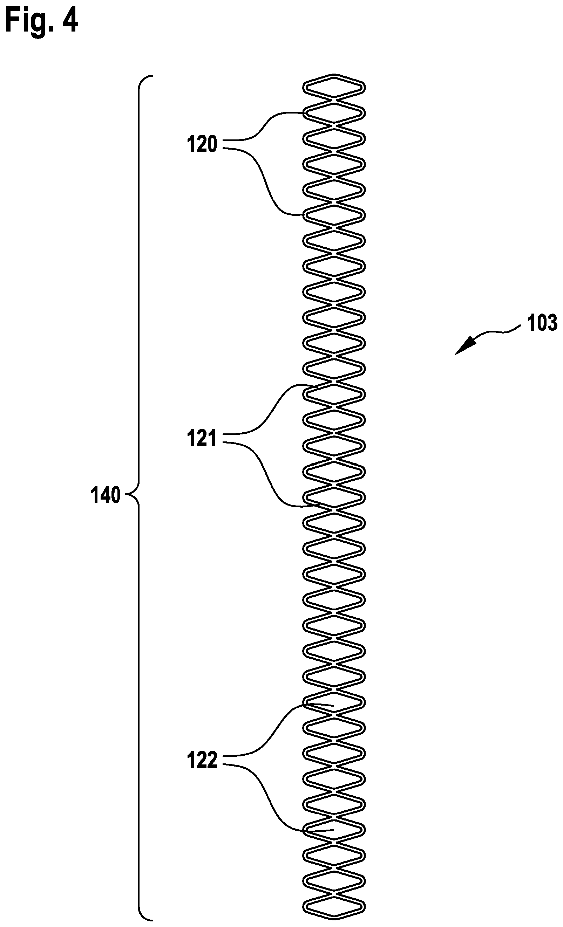

Figur 4 zeigt ein Beispiel eines nicht in den Schutzbereich der Ansprüche fallenden Stabilisierungsstabs 103.

Der Stabilisierungsstabs 103 besteht nur aus dem biegsamen Abschnitt 140, der hier nur aus einer Gitterbandstruktur 120 gebildet. Die Gitterbandstruktur 120 wird wieder aus einem Gitter 121 gebildet, das rautenförmige Zwischenräume 122 umschließt. Durch die Gitterbandstruktur 120 ist der Stabilisierungsstab 103 biegsam und leicht.

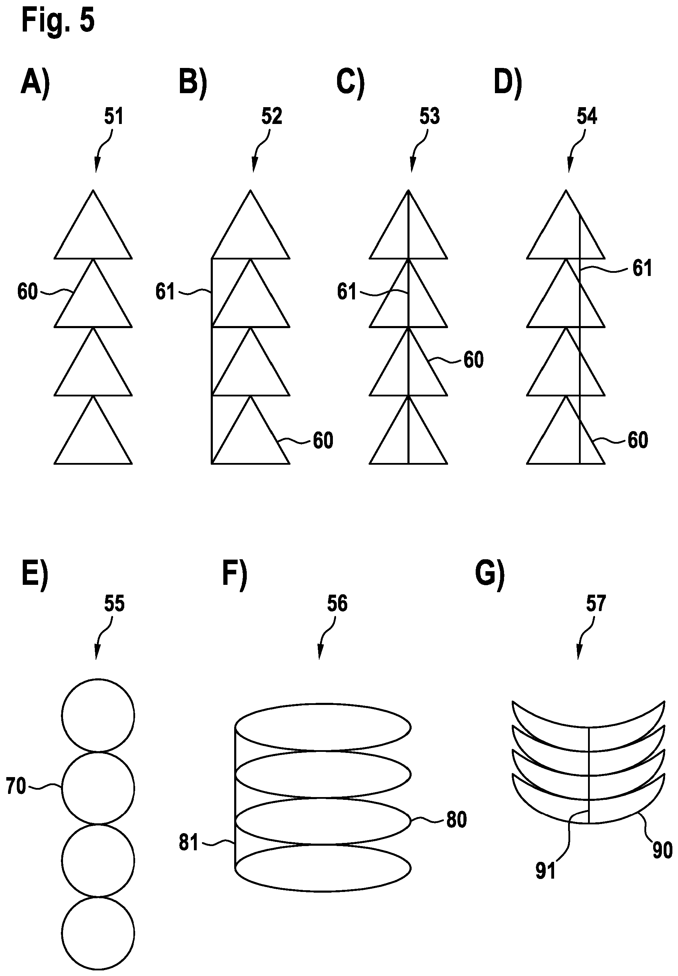

Figur 5 zeigt verschiedene Ausführungsformen der Gitterbandstruktur 51, 52, 53, 54, 55,56,57, die alternativ zu der in den Figuren 1 bis 4 gezeigten Rautenstruktur verwendet werden können.

Es ist jeweils ein Ausschnitt mit vier Strukturelementen gezeigt. In den Figuren 5A, 5B, 5C und 5D sind die Strukturelemente als Dreiecke 60 ausgebildet. Die Gitterbandstruktur 52, 53, 54 der Figuren 5B, 5C und 5D hat zusätzlich noch einen dünnen biegsamen Stab 61, der in den drei Ausführungsformen unterschiedlich positioniert ist. In Figur 5E sind die Strukturelemente der Gitterbandstruktur 55 als Kreise 70 ausgebildet. In Figur 5F sind die Strukturelemente der Gitterbandstruktur 56 als Ovale 80 ausgebildet, denen wieder ein dünner biegsamer Stab 81 zugeordnet ist. In Figur 5G sind die Strukturelemente der Gitterbandstruktur 57 als länglich gekrümmte Körper 90 ausgebildet, denen auch ein dünner biegsamer Stab 91 zugeordnet ist. Die äußeren Enden der gekrümmten Körper 90 können beim Biegen des Stabs aneinanderstoßen und so als Anschlagsbegrenzung funktionieren.

## Patentansprüche

1. Stabilisierungsstab (99,101) für ein orthopädisches Hilfsmittel (200), wobei der Stabilisierungsstab (99,101) einen biegsamen Abschnitt (140) aufweist, wobei mindestens ein Ende des Stabilisierungsstabs (99, 101) ein Griffstück (111a, 111b) aufweist, wobei der biegsame Abschnitt (140) eine Gitterbandstruktur (120) aufweist, **dadurch gekennzeichnet, dass** der Stabilisierungsstab (99,101) einstückig ist und aus einem Kunststoff besteht, wobei der Stabilisierungsstab (99,101) einen dünnen biegsamen Stab (150) und mindestens eine an diesem dünnen biegsamen Stab verlaufende Gitterbandstruktur (120, 130) aufweist oder aus zwei Gitterbandstrukturen (120,130) und einem zwischen diesen verlaufenden dünnen biegsamen Stab (150) gebildet wird.

2. Stabilisierungsstab (99,101) nach Anspruch 1, wobei Strukturelemente der Gitterbandstruktur (120,130) nicht mehr als vier Ecken aufweisen.

3. Stabilisierungsstab (99,101) nach einem der vorhergehenden Ansprüche, wobei sich die Gitterbandstruktur (120) über die Länge des Stabilisierungsstabs (99,101) erstreckt.

4. Stabilisierungsstab (99,101) nach einem der vorhergehenden Ansprüche, wobei der Stabilisierungsstab aus einem dünnen biegsamen Stab (150) und mindestens einer an diesem dünnen biegsamen Stab verlaufenden Gitterbandstruktur (120, 130) gebildet wird.

5. Stabilisierungsstab (99,101) nach einem der vorhergehenden Ansprüche, wobei der dünne biegsame Stab (150) einen Führungskanal aufweist.

6. Stabilisierungsstab (101) nach einem der vorhergehenden Ansprüche, wobei der Stabilisierungsstab (101) aus zwei Gitterbandstrukturen (120, 130) und bevorzugt einem zwischen diesen verlaufenden dünnen biegsamen Stab (150) gebildet wird.

7. Stabilisierungsstab (99,101) nach einem der vorhergehenden Ansprüche, wobei es sich bei dem orthopädischen Hilfsmittel um eine Bandage handelt, insbesondere um eine Kniegelenkbandage (200).

8. Orthopädisches Hilfsmittel, umfassend einen Stabilisierungsstab (99,101) nach einem der vorhergehenden Ansprüche.

9. Orthopädisches Hilfsmittel nach Anspruch 8, wobei das orthopädische Hilfsmittel eine Kniegelenksbandage (200) ist.

10. Kniegelenksbandage (200) nach Anspruch 9, wobei die Kniegelenksbandage (200) zwei Stabilisierungsstäbe (99,101, 102) nach einem der Ansprüche 1 bis 7 aufweist, wobei sich die Stabilisierungsstäbe (99, 101, 102) über die Länge der Kniegelenksbandage (200) erstrecken.

11. Kniegelenksbandage (200) nach Anspruch 10, wobei sich jeweils der biegsame Abschnitt (130) der Stabilisierungsstäbe (99, 101, 102) im angelegten Zustand der Kniegelenksbandage (200) zumindest auf der Höhe des Knies (301) befindet.

## Claims

1. A stabilizing rod (99,101) for an orthopedic aid (200), wherein the stabilizing rod (99,101) has a bending section (140), wherein at least one end of the stabilizing rod (99,101) has a gripping piece (111a,111b), wherein the bending section (140) has a mesh belt structure (120), **characterized in that** the stabilizing rod (99,101) is designed as one piece and consists of a plastic, wherein the stabilizing rod (99,101) has a thin bending rod (150) and at least one mesh belt structure (120, 130) running on said thin bending rod or is formed from two mesh belt structures (120,130) and one thin bending rod (150) running between them.

2. The stabilizing rod (99,101) according to claim 1, wherein structural elements of the mesh belt structure (120,130) have no more than four corners.

3. The stabilizing rod (99,101) according to one of the previous claims, wherein the mesh belt structure (120) extends over the length of the stabilizing rod (99,101).

4. The stabilizing rod (99,101) according to one of the previous claims, wherein the stabilizing rod is formed from a thin bending rod (150) and at least one mesh belt structure (120, 130) running on said thin bending rod.

5. The stabilizing rod (99,101) according to one of the previous claims, wherein the thin bending rod (150) has a guide channel.

6. The stabilizing rod (101) according to one of the previous claims, wherein the stabilizing rod (101) is formed from two mesh belt structures (120, 130) and preferably one thin bending rod (150) running between them.

7. The stabilizing rod (99,101) according to one of the previous claims, wherein the orthopedic aid is a bandage, particularly a knee joint bandage (200).

8. An orthopedic aid, comprising a stabilizing rod (99,101) according to one of the previous claims.

9. The orthopedic aid according to claim 8, wherein the orthopedic aid is a knee joint bandage (200).

10. The knee joint bandage (200) according to claim 9, wherein the knee joint bandage (200) has two stabilizing rods (99,101, 102) according to one of claims 1 to 7, the stabilizing rods (99, 101, 102) extending over the length of the knee joint bandage (200).

11. The knee joint bandage (200) according to claim 10, wherein each of the bending sections (130) of the stabilizing rods (99, 101, 102) is located at least at the level of the knee (301) when the knee joint bandage (200) is in the applied state.

## Revendications

1. Une barre stabilisatrice (99,101) destinée à un auxiliaire orthopédique (200), la barre stabilisatrice (99,101) ayant une section de flexion (140), au moins une extrémité de la barre stabilisatrice (99,101) ayant une pièce de préhension (111a,111b), la section de flexion (140) ayant une structure de bande maillée (120), **caractérisée en ce que** la barre stabilisatrice (99,101) est formée en une seule pièce et consiste en une matière plastique, la barre stabilisatrice (99,101) ayant une barre de flexion mince (150) et au moins une structure de bande maillée (120,130) courant sur ladite barre de flexion mince, ou étant formée de deux structures de bande maillée (120,130) et d'une barre de flexion mince (150) courant entre celles-ci.

2. La barre stabilisatrice (99,101) selon la revendication 1, dans laquelle des éléments structurels de la structure de bande maillée (120,130) n'ont pas plus de quatre angles.

3. La barre stabilisatrice (99,101) selon l'une des revendications précédentes, dans laquelle la structure de bande maillée (120) s'étend sur la longueur de la barre stabilisatrice (99,101).

4. La barre stabilisatrice (99,101) selon l'une des revendications précédentes, dans laquelle la barre stabilisatrice est formée d'une barre de flexion mince (150) et d'au moins une structure de bande maillée (120,130) courant sur ladite barre de flexion mince.

5. La barre stabilisatrice (99,101) selon l'une des revendications précédentes, dans laquelle la barre de flexion mince (150) a un canal de guidage.

6. La barre stabilisatrice (101) selon l'une des revendications précédentes, dans laquelle la barre stabilisatrice (101) est formée de deux structures de bande maillée (120,130) et, préférablement, d'une barre de flexion mince (150) courant entre celles-ci.

7. La barre stabilisatrice (99,101) selon l'une des revendications précédentes, dans laquelle l'auxiliaire orthopédique est un bandage, particulièrement un bandage de l'articulation du genou (200).

8. Un auxiliaire orthopédique, comprenant une barre stabilisatrice (99,101) selon l'une des revendications précédentes.

9. L'auxiliaire orthopédique selon la revendication 8, dans lequel l'auxiliaire orthopédique est un bandage de l'articulation du genou (200).

10. Le bandage de l'articulation du genou (200) selon la revendication 9, dans lequel le bandage de l'articulation du genou (200) a deux barres stabilisatrices (99,101, 102) selon l'une des revendications 1 à 7, les barres stabilisatrices (99,101, 102) s'étendant sur la longueur du bandage de l'articulation du genou (200).

11. Le bandage de l'articulation du genou (200) selon la revendication 10, dans lequel chacune des sections de flexion (130) des barres stabilisatrices (99,101,102) est située au moins au niveau du genou (301) lorsque le bandage de l'articulation du genou (200) est à l'état appliqué.
